(19) Europäisches Patentamt

European Patent Office

Office européen des brevets

(11) **EP 1 538 979 B1**

(12) **EUROPEAN PATENT SPECIFICATION**

(45) Date of publication and mention
of the grant of the patent:
**22.03.2006 Bulletin 2006/12**

(51) Int Cl.:
***A61B 5/053*** (2006.01)   ***A61N 1/368*** (2006.01)

(21) Application number: **03765413.4**

(22) Date of filing: **23.06.2003**

(86) International application number:
**PCT/SE2003/001081**

(87) International publication number:
**WO 2004/008959 (29.01.2004 Gazette 2004/05)**

(54) **A CONGESTIVE HEART FAILURE MONITOR**

ÜBERWACHUNGSGERÄT FÜR DEKOMPENSIERTE HERZINSUFFIZIENZ

MONITEUR D'INSUFFISANCE CARDIAQUE CONGESTIVE

(84) Designated Contracting States:
**CH DE FR IE IT LI**

(30) Priority: **22.07.2002 SE 0202289**

(43) Date of publication of application:
**15.06.2005 Bulletin 2005/24**

(73) Proprietor: **St Jude Medical AB**
**175 84 Järfälla (SE)**

(72) Inventors:
• **HOLMSTRÖM, Nils**
**S-175 49 Järfälla (SE)**
• **OBEL, Martin**
**S-182 33 Danderyd (SE)**

• **NORLIN, Anna**
**S-114 22 Stockholm (SE)**
• **DAHLBERG, Kenneth**
**S-112 26 Stockholm (SE)**
• **BJÖRLING, Anders**
**S-175 53 Järfälla (SE)**
• **KALLING, Sven**
**S-183 56 Täby (SE)**
• **LJUNGSTRÖM, Karin**
**S-165 70 Hässelby (SE)**
• **NOREN, Kjell**
**S-171 58 Solna (SE)**

(56) References cited:
EP-A2- 0 985 429     US-A- 4 674 518
US-B1- 6 223 079     US-B1- 6 278 894

## Description

### *Technical field*

The present invention relates to a congestive heart failure monitor.

### *Background*

Electrical stimulation therapy of congestive heart failure is previously known. Thus in US 5 584 868 a dual-chamber pacemaker designed for treating congestive heart failure (CHF) by changing the AV interval is described and in US 6 223 079 B1 which discloses the features of the preamble of claim 1, a four chamber pacing system for improving cardiac output of CHF patients by controlling pacing to maintain the ventricular mechanical synchronization is disclosed. For providing suitable timing in the latter system impedance sensing in the left heart is used.

Incipient CHF is often present without the patient knowing it. An indicator for incipient CHF would therefore be of great value since treatment by addition of drugs or electrical stimulation therapy could then be introduced at an early stage of CHF to slow down the progression of CHF. This would prolong the survival of the patient. Such an indicator could also be used to alert the patient or the physician about new conditions so appropriate measures can be taken. The first sign of a CHF can be seen in the left atrium, for instance in volume changes thereof.

The purpose of the present invention is to utilize this knowledge to provide a congestive heart failure monitor for detecting CHF at an early stage.

### *Disclosure of the invention*

This purpose is obtained by a congestive heart failure monitor acoording to claim 1.

The first sign of a CHF can be observed in the left atrium of the heart by monitoring its mechanical behaviour, like volume changes, as mentioned above. If the pumping ability of the left ventricle is reduced the volume of the left atrium will increase due to the excessive filling of blood. The filing pattern of the left atrium can be disturbed due to mitral regurgitation caused by either diastolic or systolic dysfunction. The diastolic dysfunction could be a result of prolonged PR interval, i.e. the P-wave to QRS interval or too long an AV interval, resulting in reversed flow back to the left atrium during diastole as the mitral valve does not close immediately after the atrial contraction. The systolic dysfunction could be a result of infarctic areas in the left ventricle which disturbs the left ventricle contraction propagation so that the mitral valve cannot close properly, (the papillar muscle get asynchronous), bringing reversed flow back to the left atrium during systole. The systolic dysfunction in the left ventricle could also be a result of bad timing of the right and left ventricle stimulations (e.g. septum, innervated at RVOT stimulation, is involved in the left ventricle contraction) causing mitral regurgitation and disturbed filling pattern of the left atrium. All these conditions results in a disturbed filling pattern of the left atrium and is one of the first signs of CHF.

A first sign of CHF can thus be observed in the left atrium and since the conductivity of blood is different from that of tissue the monitor according to the invention comprises an impedance measuring unit adapted to measure impedance between at least two electrodes intended to be implanted in the patient such that a change in the left atrium volume results in a change in the measured impedance. In this way not only incipient CHF can be detected but the monitor according to the invention can also be used as a diagnostic tool for studying the progression or regression of CHF for enabling proper treatment of the patient.

According to advantageous embodiments of the monitor according to the invention the analyzing means comprise an averaging means provided to form a mean value of the measured impedance during a plurality of cardiac cycles and the analyzing means are adapted to analyze the mean value to detect CHF or said analyzing means comprise a quotient determining means provided to determine the quotient between the impedance minimum and maximum values during a cardiac cycle, and the analyzing means are adapted to analyze the quotient to detect CHF. Preferably the analyzing means are adapted to analyze both the impedance mean value and the quotient to detect CHF. Firstly, even though the impedance changes continuously during the heartbeat, the mean value will decrease when the left atrium volume increases. Secondly, the quotient between the impedance minimum and maximum values will be larger, with increasing blood filling of the left atrium. Accordingly with the present invention an efficient CHF monitor is provided based on the analysis of these two quantities.

According to other advantageous embodiments of the monitor according to the invention the electrodes are designed for implantation in the right and left atria, respectively, or for implantation in the right atrium and left ventricle. In case of an implantable monitor, one of the electrodes can be designed for implantation in the left atrium and the other electrode be formed of the outer capsule of the monitor, e.g. the pacemaker capsule when the monitor is included in a pacemaker.

Also other combinations of the above mentioned electrodes can be used for the impedance determination.

The electrodes intended for implantation in the left atrium and the left ventricle are preferably designed for implantation in a coronary vein. For all these alternatives signals corresponding to the blood filling of the left atrium are obtained from the electrodes.

According to still other advantageous embodiments of the monitor according to the invention the impedance measuring unit comprises a measuring circuit in the form of synchronous demodulator for obtaining both the real and imaginary parts of the impedance, and the impedance measuring unit is preferably adapted to determine the impedance phase angel for detecting and the analysing means is adapted to analyse the phase angle for detecting an incipient CHF. Since blood is resistive a high degree of blood filling results in a small phase angle. On the contrary, if more heart tissue is present, like in case of a healthy heart, the phase angle will get a larger negative value.

## Brief description of the drawings

To explain the invention in greater detail embodiments of the monitor according to the invention chosen as examples will be described below with reference to the enclosed drawings, on which figure 1 illustrates principally the impedance measurements performed in one embodiment of the monitor according to the invention, figure 2 is a simplified illustration of an embodiment of the monitor according to the invention, figure 3-5 illustrate alternative ways of performing impedance unit of the monitor according to the invention, and figure 7 is a flow chart illustrating the signal processing in an embodiment of the monitor according to the invention.

## Detailed description of embodiments

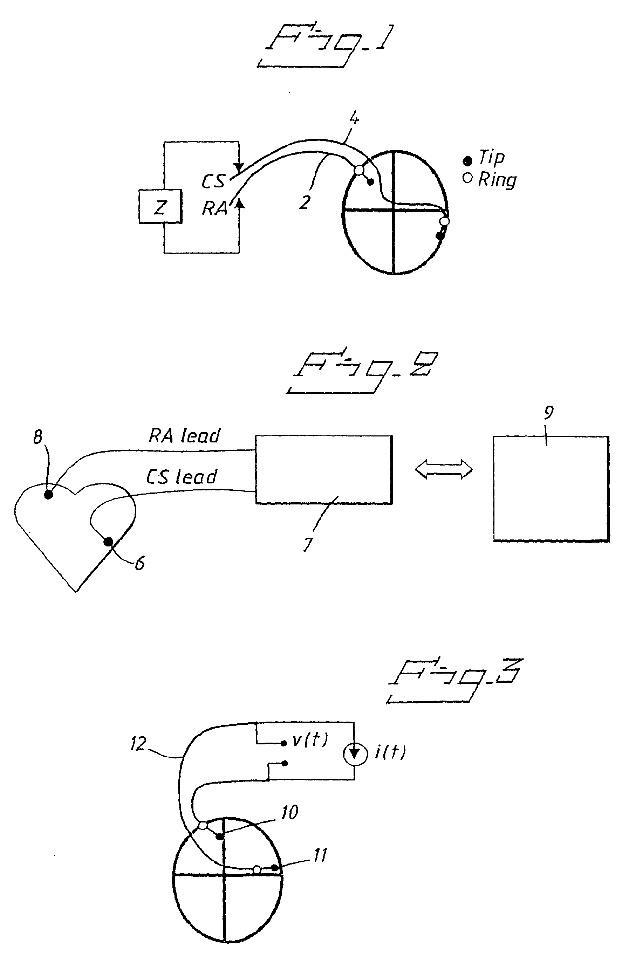

Figure 1 illustrates principally measurement of the impedance Z between the right atrial lead 2 and the coronary sinus lead 4. As the left atrium is dilated due to CHF the impedance Z will decrease. Also the variation of the impedance between maximum and minimum values will then decrease due to increased wall tension.

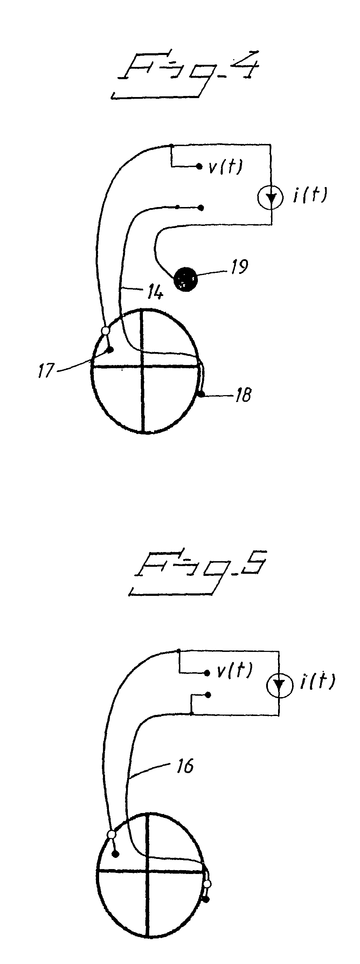

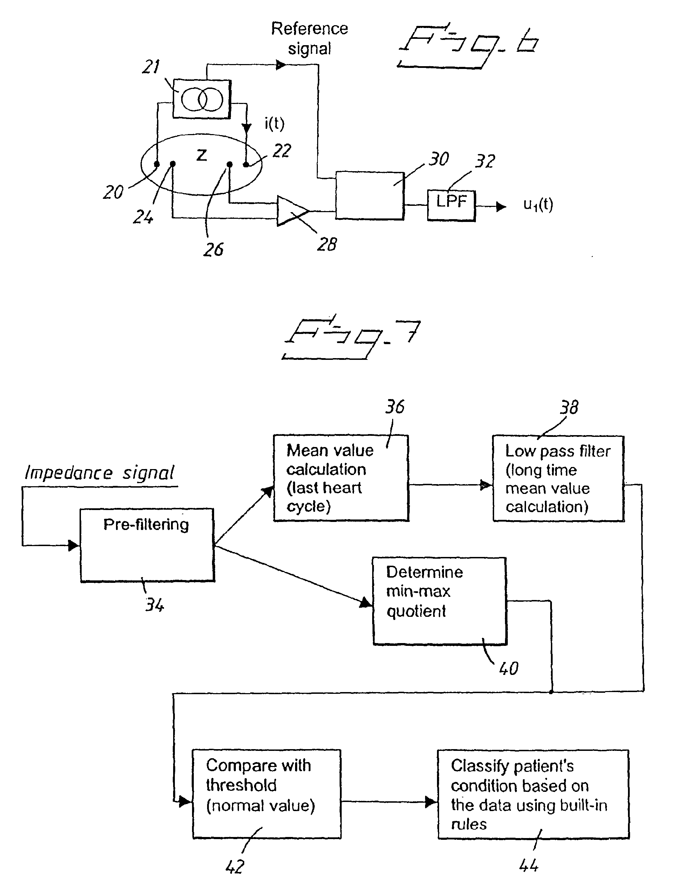

To secure a safe fixation of the left atrial electrode 6 in the coronary sinus CS or the great cardiac vein it is beneficial to use a screw-in electrode, cf. figure 2. The optimal right atrial RA electrode 2 position is lightly to be in the inter-atrial septum near the coronary sinus ostium, see the electrode tip 10 in figure 3. With the electrodes 6, 8; 10,11 positioned as shown in figures 2 and 3 the volume of the left atrium is positioned between the electrodes. This enables variations of impedance variations across the left atrium and a high sensitivity to left atrium volume changes.

Also other bipolar electrode measurements set-ups as well as tripolar electrode settings are possible in the monitor according to the invention.

The embodiment of the monitor according to the invention shown in figure 2 comprises monitor electronics 7 for analysis of the measured impedance for detection of an incipient CHF. An implantation monitor is preferably also provided with telemetry means, not shown in figure 2, for communication with an external programmer and data acquisition device 9.

Figures 3-5 illustrate quadropolar electrode configurations suitable for use in the monitor according to the invention. In figure 3 the coronary sinus CS lead 12 is positioned on the left atrium and in the figures 4 and 5 the CS lead 14 and 16 respectively is placed on the left ventricle.

The method of bio-impedance measurement is not critical in the monitor according to the invention. Figures 3-5 illustrate a technique wherein an electric current i(t) is supplied between two electrodes and the resulting evoked voltage response v(t) is detected. In the embodiments shown in figures 3 and 5 the evoked voltage response i(t) is supplied. Figure 4 shows an embodiment in which the current i(t) is supplied between a right atrial electrode 17 and a stimulator can 19, whereas the evoked voltage response is measured between the right atrial electrode 17 and a left ventricular electrode 18 positioned in the coronary sinus.

Figure 6 shows an alternative embodiment of the impedance measuring unit of the monitor according to the invention in the form of a synchronous demodulator. Generally the electric current i(t) is applied to two electrodes 20, 22 and the resulting evoked response is measured between two measurement electrodes 24 and 26. The measured voltage signal is amplified in an amplifier 28. The measured voltage signal is synchronized with the current i(t) with the aid of a reference signal picked up from the current source 21 and supplied to synchronizing means in the form of multiplier 30. A low-pass filter 32 is provided to filter the output signal from the multiplier 30. The resulting impedance $Z_1$ is the given by the expression

$$Z_1 = u_1 / i$$

where $u_1$ denotes the filtered resulting synchronized output voltage signal.

With the impedance measuring circuit shown in figure 6 both the real and the imaginary parts of the impedance are measured and consequently the impedance phase angle is obtained too.

As discussed above, at left ventricular dysfunction the left atrium will dilate according to the progress of the disease, because the left ventricle is not able to eject blood into the body and blood will consequently stagnate in the left atrium and pulmonary veins. Left atrium blood pressure will increase as well as left atrium wall tension. The blood volume in the left atrium will also increase while the variation between maximum and minimum volume values will decrease. These phenomena can be determined from the measured impedance.

Figure 7 is a flow chart illustrating an example of an embodiment of the monitor according to the invention analysing the impedance minimum-maximum quotient and the overall impedance mean value for detecting an early CHF. The impedance raw signal obtained as explained above is pre-filtered, at 34 in the figure. The filtering at 34 is performed to remove artefacts of noise, breathings etc. Mean value of the impedance signal during the last heart cycle is calculated in averaging means, at 36, and long time mean value calculation is performed by means of a low pass filter, at 38. The expression "long time" could mean a time of the order of typically 10 minutes in this connection.

At 40 in figure 7 the quotient between the impedance minimum and maximum values is determined. The obtained long term mean value and the quotient between minimum and maximum values are compared with predetermined reference or normal threshold values in comparison means, at 42 in the figure. The results of these comparisons are used, at 44, to classify the patient's condition according to predetermined built-in rules.

The processing described above with reference to figure 7 can advantageously be used together with an activity sensor and a posture sensor. The impedance properties can then be calculated during the same conditions for the patient, for instance with the patient in a resting supine position. The processing chain of figure 7 can also preferably contain a memory for saving the time history of calculated parameters for further evaluation in external devices, cf. figure 2.

## Claims

1. A congestive heart failure monitor comprising an impedance measuring unit (7; 30, 32) adapted to measure the impedance Z between at least two electrodes (2, 4; 6, 8; 10, 12; 17, 18; 20, 22, 24, 26) intended to be implanted in a patient such that a change in the left atrium volume results in a change of the measured impedance, and analysing means (7, 9; 36, 38, 40, 42, 44) for analysing said measured impedance for detecting an incipient congestive heart failure (CHF) **characterized in that** said analysing means comprise a quotient determining means (40) provided to determine the quotient between the impedance minimum and maximum values during a cardiac cycle, and **in that** said analysing means are adapted to analyse said quotient to detect CHF.

2. The monitor according to claim 1, **characterized in that** said analysing means comprise an averaging means (36, 38) provided to form a mean value of said measured impedance during a plurality of cardiac cycles, and **in that** said analysing mans are adapted to analyse said mean value to detect CHF.

3. The monitor according to claim 2, **characterized in that** a first comparison means is provided to compare said impedance mean value with a predetermined impedance threshold value, said analysing means being adapted to detect CHF from the result of said comparison.

4. The monitor according to claim 1, **characterized in that** said analysing means comprise quotient averaging means provided to form a mean value of said quotient during a plurality of cardiac cycles, and **in that** said analysing means are adapted to analyse said mean value to detect CHF.

5. The monitor according to claims 1 or 4, **characterized in that** a second comparison means is provided to compare said quotient or said mean value of the quotient with a predetermined quotient threshold value, said analysing means being adapted to detect CHF from the result of said comparison.

6. The monitor according to claim 3, **characterized in that** said averaging means is adapted to form a floating mean value of the measured impedance during a predetermined number of preceding cardiac cycles for use as said impedance threshold value.

7. The monitor according to claim 5, **characterized in that** said quotient averaging means are adapted to form a floating mean value of said quotient determined during a predetermined number of preceding cardiac cycles for use as said quotient threshold value.

8. The monitor according to any of the claims 1-7, **characterized in that** said analysing means are adapted to analyse said impedance mean value and said quotient to detect CHF.

9. The monitor according to claims 3 and 5, **characterized in that** said first and second comparison means are one and the same comparison means (42).

10. The monitor according to any of the preceding claims, **characterized in that** said electrodes (6, 8; 10, 12) are designed for implantation in the right and left atria, respectively.

11. The monitor according to any of the claims 1-9, **characterized in that** said electrodes (2, 4; 17, 18) are designed for implantation in the right atrium and left ventricle.

12. The monitor according to any of the claims 1- 9, said monitor being implantable, **characterized in that** one of said electrodes (18) is designed for implantation in the left atrium and the other electrode is formed of an outer capsule of the monitor.

13. The monitor according to any of the claims 10 - 12, **characterized in that** said electrodes (12; 18; 16) for implantation in the left atrium and the left ventricle are designed for implantation in a coronary vein.

14. The monitor according to claim 1, **characterized in that** said impedance measuring unit comprises a measuring circuit in the form of a synchronous demodulator for obtaining both the real and imaginary parts of the impedance (fig. 6).

15. The monitor according to claim 14, **characterized in that** said impedance measuring unit is adapted to determine the impedance phase angle and **in that** said analysing means are adapted to analyse the phase angle for detecting an incipient CHF.

16. A multisite heart stimulator, **characterized by** a monitor according to any one of the preceding claims.

17. The stimulator according to claim 16, **characterized by** a control unit provided to control the stimulation of the patient's heart in response to an output signal received from said monitor and representing the results of the analysis of the measured impedance, in order to optimize hemodynamics.

**Patentansprüche**

1. Überwachungsgerät für kongestive Herzinsuffizienz, enthaltend eine Impedanzmesseinheit (7; 30, 32), die ausgelegt ist, die Impedanz Z zwischen wenigstens zwei Elektroden (2, 4; 6, 8; 10, 12; 17, 18; 20, 22, 24, 26) zu messen, welche vorgesehen sind, in einem Patienten so implantiert zu werden, dass eine Änderung im Volumen des linken Atriums zu einer Änderung der Messimpedanz führt, und enthaltend eine Analysevorrichtung (7, 9; 36, 38; 40, 42, 44) zum Analysieren der genannten gemessenen Impedanz für die Erfassung einer beginnenden kongestiven Herzinsuffizienz (CHF), **dadurch gekennzeichnet, dass** die genannte Analysevorrichtung eine Quotientenbestimmungsvorrichtung (40) enthält, die vorgesehen ist, während eines Herzzyklus den Quotienten zwischen dem Minimalwert und dem Maximalwert der Impedanz zu bestimmen, und dass die genannte Analysevorrichtung ausgelegt ist, den genannten Quotienten zu analysieren, um die CHF zu erfassen.

2. Überwachungsgerät nach Anspruch 1, **dadurch gekennzeichnet, dass** die Analysevorrichtung eine Mittelwertbildungsvorrichtung (36, 38) enthält, die vorgesehen ist, um einen Mittelwert aus der genannten, während einer Vielzahl von Herzzyklen gemessenen Impedanz zu bilden, und dass die genannte Analysevorrichtung ausgelegt ist, den genannten Mittelwert zu analysieren, um die CHF zu detektieren.

3. Überwachungsgerät nach Anspruch 2, **dadurch gekennzeichnet, dass** eine erste Vergleichsvorrichtung vorgesehen ist, um den genannten Impedanzmittelwert mit einem vorbestimmten Impedanzschwellenwert zu vergleichen, wobei die genannte Analysevorrichtung ausgelegt ist, die CHF aus dem Ergebnis des genannten Vergleichs zu detektieren.

4. Überwachungsgerät nach Anspruch 1, **dadurch gekennzeichnet, dass** die genannte Analysevorrichtung eine Quotientenmittelwertsbildungsvorrichtung enthält, die vorgesehen ist, um einen Mittelwert des genannten Quotienten während einer Vielzahl von Zyklen zu bilden, und dass die genannte Analysevorrichtung ausgelegt ist den genannten Mittelwert zu analysieren, um die CHF zu detektieren.

5. Überwachungsgerät nach Anspruch 1 oder 4, **dadurch gekennzeichnet, dass** eine zweite Vergleichsvorrichtung vorgesehen ist, um den genannten Quotienten oder den genannten Mittelwert des Quotienten mit einem vorbestimmten Quotientenschwellenwert zu vergleichen, wobei die genannte Analysevorrichtung ausgelegt ist, die CHF aus dem Ergebnis des genannten Vergleichs zu detektieren.

6. Überwachungsgerät nach Anspruch 3, **dadurch gekennzeichnet, dass** die genannte Mittelwertbildungsvorrichtung ausgelegt ist, während einer vorbestimmten Anzahl von vorhergehenden Herzzyklen einen fließenden Mittelwert der gemessenen Impedanz zur Verwendung als den genannten Impedanzschwellenwert zu bilden.

7. Überwachungsgerät nach Anspruch 5, **dadurch gekennzeichnet, dass** die genannte Quotienten-Mittelwertbildungsvorrichtung ausgelegt ist, von dem genannten, während einer vorbestimmten Anzahl vorhergehender Herzzyklen bestimmten Quotienten einen fließenden Mittelwert zur Verwendung als den Quotienten-Schwellenwert zu bilden.

8. Überwachungsgerät nach einem der Ansprüche 1 bis 7, **dadurch gekennzeichnet, dass** die genannte Analysevorrichtung ausgelegt ist, den genannten Impedanzmittelwert und den genannten Quotienten zu analysieren, um die CHF zu detektieren.

9. Überwachungsgerät nach den Ansprüchen 3 und 5, **dadurch gekennzeichnet, dass** die genannte erste und die genannte zweite Vergleichsvorrichtung ein- und dieselbe Vergleichsvorrichtung (42) sind.

10. Überwachungsgerät nach einem der vorhergehenden Ansprüche, **dadurch gekennzeichnet, dass** die genannten Elektroden (6, 8; 10, 12) für eine Implantation in das rechte bzw. in das linke Atrium ausgebildet sind.

11. Überwachungsgerät nach einem der Ansprüche 1 bis 9, **dadurch gekennzeichnet, dass** die genannten Elektroden (2, 4; 17, 18) für eine Implantation in das rechte Atrium und in den linken Ventrikel ausgebildet sind.

12. Überwachungsgerät nach einem der Ansprüche 1 bis 9, wobei das Überwachungsgerät implantierbar ist, **dadurch gekennzeichnet, dass** eine der genannten Elektroden (18) für die Implantation in das linke Atrium ausgebildet ist und die andere Elektrode aus einer äußeren Kapsel des Überwachungsgeräts gebildet ist.

13. Überwachungsgerät nach einem der Ansprüche 10 bis 12, **dadurch gekennzeichnet, dass** die genannten, zur Implantation in das linke Atrium und in den linken Ventrikel vorgesehenen Elektroden (12; 18; 16) für eine Implantation in eine Koronarvene ausgebildet sind.

14. Überwachungsgerät nach Anspruch 1, **dadurch gekennzeichnet, dass** die genannte Impedanzmesseinheit eine Messschaltung in Form eines synchronen Demodulators enthält, um sowohl den realen als auch den imaginären Anteil der Impedanz zu erhalten (Fig. 6).

15. Überwachungsgerät nach Anspruch 14, **dadurch gekennzeichnet, dass** die genannte Impedanzmesseinheit ausgelegt ist den Impedanzphasenwinkel zu bestimmen, und dass die genannte Analysevorrichtung ausgelegt ist, den Phasenwinkel für die Detektion einer beginnenden CHF zu analysieren.

16. Multisite-Herzstimulator, **gekennzeichnet durch** ein Überwachungsgerät nach einem der vorhergehenden Ansprüche.

17. Stimulator nach Anspruch 16, **gekennzeichnet durch** eine Steuereinheit, die vorgesehen is, die Stimulation des Herzens eines Patienten in Reaktion auf ein Ausgangssignal zu steuern, das aus dem Überwachungsgerät empfangen worden ist, und die Ergebnisse der Analyse der gemessenen Impedanz repräsentiert, um die Hämodynamik zu optimieren.

**Revendications**

1. Moniteur d'insuffisance cardiaque congestive comprenant une unité (7) ; 30, 32) de mesure d'impédance conçue pour mesure l'impédance Z entre au moins deux électrodes (2,4 ; 6, 8 ; 10, 12 ; 17, 18 ; 20, 22, 24, 26) destinées à être implantées dans un patient de sorte qu'un changement du volume de l'oreillette gauche se traduit par un changement de l'impédance mesurée et des moyens (7, 9 ; 36, 38, 40, 42, 44) d'analyse de l'impédance mesurée pour détecter une défaillance cardiaque congestive débutante (CHF), **caractérisé en ce que** les moyens d'analyse comprennent des moyens (40) de détermination d'un quotient destinés à déterminer le quotient entre les valeurs minimum et maximum d'impédance pendant un cycle cardiaque et **en ce que** les moyens d'analyse sont conçus pour analyser le quotient afin de détecter la CHF.

2. Moniteur suivant la revendication 1, **caractérisé en ce que** les moyens d'analyse comprennent des moyens (36, 38) pour faire une moyenne destinés à former une valeur moyenne de l'impédance mesurée pendant une pluralité de cycles cardiaque et **en ce que** les moyens d'analyse sont conçus pour analyser la valeur moyenne afin de détecter la CHF.

3. Moniteur suivant la revendication 2, **caractérisé en ce qu'**il est prévu des premiers moyens de comparaison de la valeur moyenne de l'impédance à une valeur de seuil d'impédance déterminée à l'avance, les moyens d'analyse étant conçus pour détecter la CHF à partir du résultat de la comparaison.

4. Moniteur suivant la revendication 1, **caractérisé en ce que** les moyens d'analyse comprennent des moyens pour faire la moyenne d'un quotient destinés à former une valeur moyenne du quotient pendant une pluralité de cycles cardiaques et **en ce que** les moyens d'analyse sont conçus pour analyser la valeur moyenne afin de détecter la CHF.

5. Moniteur suivant les revendications 1 ou 4, **caractérisé en ce qu'**il est prévu des deuxièmes moyens de comparaison pour comparer le quotient ou la valeur moyenne du quotient à une valeur de seuil de quotient déterminée à l'avance, les moyens d'analyse étant conçus pour détecter la CHF à partir du résultat de la comparaison.

6. Moniteur suivant la revendication 3, **caractérisé en ce que** les moyens pour faire la moyenne sont conçus pour former une valeur moyenne mobile de l'impédance mesurée pendant un nombre déterminé à l'avance de cycles cardiaques précédents à utiliser comme la valeur de seuil d'impédance.

7. Moniteur suivant la revendication 5, **caractérisé en ce que** les moyens pour faire la moyenne du quotient sont conçus pour former une valeur moyenne mobile du quotient déterminée pendant un nombre déterminé à l'avance de cycles cardiaques précédents à utiliser comme la valeur de seuil de quotient.

**8.** Moniteur suivant l'une quelconque des revendications 1 à 7, **caractérisé en ce que** les moyens d'analyse sont conçus pour analyser la valeur moyenne de l'impédance et du quotient pour détecter la CHF.

**9.** Moniteur suivant les revendications 3 et 5, **caractérisé en ce que** les premiers et deuxièmes moyens de comparaison sont un seul et même moyen (42) de comparaison.

**10.** Moniteur suivant l'une quelconque des revendications précédentes, **caractérisé en ce que** les électrodes (6, 8 ; 10, 12) sont conçues pour être implantées dans l'oreillette droite et gauche, respectivement.

**11.** Moniteur suivant l'une quelconque des revendications 1 à 9, **caractérisé en ce que** les électrodes (2, 4 ; 17, 18) sont conçues pour être implantées dans l'oreillette droite et le ventricule gauche.

**12.** Moniteur suivant l'une quelconque des revendications 1 à 9, le moniteur pouvant être implanté, **caractérisé en ce que** l'une des électrodes (18) est conçue pour une implantation dans l'oreillette gauche et l'autre électrode est formée d'une capsule extérieure du moniteur.

**13.** Moniteur suivant l'une quelconque des revendications 10 à 12, **caractérisé en ce que** les électrodes (12 ; 18 ; 16) à implanter dans l'oreillette gauche et le ventricule gauche sont conçues pour une implantation dans une veine coronaire.

**14.** Moniteur suivant la revendication 1, **caractérisé en ce que** l'unité de mesure d'impédance comprend un circuit de mesure sous la forme d'un démodulateur synchrone pour obtenir à la fois les parties réelle et imaginaire de l'impédance (figure 6).

**15.** Moniteur suivant la revendication 14, **caractérisé en ce que** l'unité de mesure d'impédance est conçue pour déterminer l'angle de phase de l'impédance et **en ce que** les moyens de l'analyse sont conçus pour analyser l'angle de phase afin de détecter une CFH débutante.

**16.** Stimulateur cardiaque multisites, **caractérisé par** un moniteur suivant l'une quelconque des revendications précédentes.

**17.** Stimulateur suivant la revendication 16, **caractérisé par** une unité de commande destinée à commander la stimulation du coeur du patient en réponse à un signal de sortie reçu du moniteur et représentant les résultats de l'analyse de l'impédance mesurée afin d'optimiser l'hémodynamique.

## Fig.1

Z    CS    4
     RA    2

• Tip
○ Ring

## Fig.2

8    RA lead    9
     CS lead
7
6

## Fig.3

12    v(t)    i(t)
10
11

Fig-4

Fig-5

Reference
signal

*Fig. 6*

21

i(t)

Z

22

20

24

26

28

30

32

LPF

$u_1(t)$

*Fig. 7*

36

38

*Impedance signal*

Mean value
calculation
(last heart
cycle)

Low pass filter
(long time
mean value
calculation)

Pre-filtering

34

Determine
min-max
quotient

40

Compare with
threshold
(normal value)

Classify patient's
condition based on
the data using built-in
rules

42

44